# EUROPEAN PATENT SPECIFICATION

(11) **EP 1 523 301 B1**
(45) Date of publication and mention of the grant of the patent: **19.03.2008**
(21) Application number: 03732166.8
(22) Date of filing: 08.07.2003
(51) Int. Cl.: A61K 9/127

(54) **DELIVERY SYSTEM FOR PHARMACEUTICAL AGENTS**
VERABREICHUNGSFORM FÜR PHARMAZEUTISCHE WIRKSTOFFE
SYSTEME D'APPORT D'AGENTS PHARMACEUTIQUES

(30) Priority: 10.07.2002 EP 02014991
(43) Date of publication of application: 20.04.2005
(73) Proprietor: Bron, Denis, 5016 Obererlinsbach (CH)
(72) Inventor: Bron, Denis, 5016 Obererlinsbach (CH)
(74) Representative: Blum, Rudolf Emil
(86) International application number: PCT/CH2003/000453
(87) International publication number: WO 2004/006891

(56) References cited:
- EP-A- 1 214 935
- DE-A- 10 056 136
- US-A- 5 366 958
- US-A- 5 618 916

## Description

This application claims the priority of European patent application 02 014 991.0, filed July 10, 2002.

### Technical Field

The present invention relates to a delivery system for pharmaceutical agents. Said delivery system comprises liposomes which comprise in their internal compartment a pharmaceutical agent to be delivered and which have linked to their external surface the cell adhesion molecule NCAM or a fragment thereof.

### Background Art

Liposomes have been widely used for the delivery of a variety of material to cells such as e.g. therapeutical drugs and DNA. In order to target the delivery of the encapsulated material to a specific cell type, various liposome modifications have been developed. In one approach adhesive peptides binding the cell surface molecule ICAM-1 (intercellular adhesion molecule-1) on the target cells were linked to the lipsome surface (Jaafari MR, Foldvari M, J. Pharm. Sci. 91(2): 396-404, 2002). In another approach cationic liposomes were targeted in vi*tro* to specific tumor cells by means of monoclonal antibodies (Kao GY et al., Cancer Gene Ther. 3(4): 250-6, 1996).

The hitherto know liposome based delivery systems have a major drawback, since they do not allow an efficient delivery of pharmaceutical agents to cells. There is therefore a need for alternative liposome based delivery systems for pharmaceutical agents.

### Disclosure of the Invention

Hence it is an object of the present invention to provide a system for the intracellular delivery of pharmaceutical agents wherein said system comprises liposomes which comprise in their internal compartment a pharmaceutical agent and which have linked to their external surface the cell adhesion molecule NCAM (neural cell adhesion molecule) or a fragment thereof.

In a preferred embodiment of the present invention said fragment of NCAM comprises IG (immunoglobulin ) loop domains I, II and III.

In a preferred embodiment of the present invention said cell adhesion molecule is linked to said external surface of said liposomes via a transmembrane domain or a hydrophobic anchor molecule which allows the cell adehsion protein to be tethered to the lipid bilayer such as e.g. a GPI anchor or by isoprenylation of the cell adhesion molecule.

Methods for the production of liposomes are known to the man skilled in the art and *e*.*g*. described in Liposomes a practical approach. RRC New Editor. IRL press at oxford university press. 1990*.*

The term "cell adhesion molecule" as used herein encompasses at least cell adhesion molecules isolated from their natural source, cell adhesion molecules produced by recombinant technology, and cell adhesion molecules produced by *in vitro* peptide synthesis and fragments thereof.

The term pharmaceutical agent as used herein encompasses all classes of chemical compounds exerting an effect in a biological system. Preferred pharmaceutical agents for the use in the present invention are molecules selected from the group consisting of DNA, RNA, oligonucleotides, polypeptides, peptides, antineoplastic agents, hormones, vitamins, enzymes, antivirals, antibiotics, antiinflammatories, antiprotozoans, antirheumatics, radioactive compounds, antibodies, prodrugs, and combinations thereof.

In a preferred embodiment of the present invention said pharmaceutical agent is a DNA, preferably a cDNA which is operably linked to a gene expression construct allowing expression of said cDNA, more preferably said cDNA encodes a functional protein. Typically, an expression construct comprises the regulatory sequences required to achieve expression in the host cell and it may contain necessary sequences required for plasmid replication in order to exist in an episomal state, or it may be designed for chromosomal integration. The term regulatory sequence as used herein encompasses both the native regulatory sequence of the gene to be expressed from said expression construct and heterolgous regulatory sequences. The construction of such expression constructs are known to the man skilled in the art and are e.g described in Sambrook et al., Molecular Cloning: A Laboratory Manual, New York: Cold Spring Harbor Laboratory, 2001.

When said expression construct is intented for chromosomal integration, the integration into the host genome is preferably achieved by including cis-elements into the expression construct that can be recognized by a transiently expressed transposase such as e.g. Sleeping Beauty. The transposase can be expressed temporarily by including the cognate mRNA into the delivery system. Said expression costruct intended for chromosomal integration preferably comprises cis-elements preventing transcriptional silencing that follows random integration.

In a much preferred embodiment said DNA construct is designed for selective integration into the genome of host cells. For this purpose, the integrase of phi bacteriophage C31 is preferably used (Ortiz-Urda S. et al., Nat Med 2002, 8 (10): 1166-70). When using the phi bacteriophage C31 integrase the DNA construct comprises the AttB sequence which is recognized by C31 integrase. The integrase can e.g. be expressed in the host cells from a adenoviral expression vector which is delivered into the cells with the inventive delivery system. It is as well possible to include into the inventive delivery system *in vitro* produced mRNA coding for C31 integrase. The transient expression of C31 integrase in host cells via a recombinant-adenovirus or via a mRNA bears on the notion that long term expression of such DNA rearranging enzymes could itself be genotoxic and therefore should be avoided.

In a another preferred embodiment said DNA construct further comprises locus control elements (LCR) e.g. matrix attachment sites.

In a much preferred embodiment of the present invention said DNA encodes the human dystrophin protein.

In a preferred embodiment of the invention said delivery system comprising as pharmaceutical agent a DNA further comprises a DNA compacting agent such as e.g.cationic peptides. Said DNA compacting agents reduce the size of the liposome particles. Preferred DNA compacting agents are reversibly cross-linkable cations that can be crosslinked after particle formation. A much preferred crosslink is a thio bridge.

In another preferred embodiment of the invention said delivery system comprising as pharmaceutical agent a DNA further comprises a chemical inclusion such as e.g. secondary and tertiary amines and/or a biological inclusion such as e.g. adeno fiber, to breach the intracellular endosomal barrier.

Said delivery system comprising as pharmaceutical agent a DNA preferably comprises nuclear localisation signals, more preferably said nuclear localisation signals are PNA linked peptides and/or PNA linked ligands. Said PNA linked peptides or PNA linked ligands can be picked up by nuclear-cytoplasmic shuttles and lead to the nuclear delivery of the DNA.

In another preferred embodiment of the invention said delivery system comprising as pharmaceutical agent a DNA further comprises an anti-apoptotic activity. Preferred anti-apoptotic activities are selected from the group consisting of Bcl-2, a small interfering RNA (siR-NA) directed against Bax and a peptid comprising caspase inhibitor sequences. The preferred anti-apoptotic activity for use with the inventive delivery system is Bcl-XL. The inventive delivery system can comprise one of said anti-apoptotic activities or a mixture thereof. Said activities are included into the inventive delivery system in order to avoid that successfully transfected cells are lost due to apoptosis. The anti-apoptotic activity can e.g. be present in the inventive delivery system in form of an adenoviral expression vector or in form of in vitro produced mRNA.

It has to be understood that the inventive delivery system for DNA can comprise any combination of the structural elements disclosed in the above embodiments.

Another object of the present invention is a pharmaceutical composition comprising a delivery system according to the present invention.

The delivery system and the pharmaceutical composition of the present invention are suitable tools for the treatment of various human diseases such as e.g. genetic diseases and cancer as well as for gene therapy. The inventive delivery system comprising as pharmaceutical agent a DNA encoding a functional dystrophin protein is a useful tool for the treatment of Duchenne muscular dystrophy. Depending on the cell adhesion molecule included in the inventive delivery system a pharmaceutical agent can be targeted to specific cells and/or tissue in the human or animal body. The delivery system of the present invention can as well be used for the introduction of pharmaceutical agents into cells *in vitro.*

The inventive delivery system or pharmaceutical composition can be administered intravenously as a bolus or by continuous infusion over a period of time, by intramuscular, subcutaneous, intra-articular, intrasynovial, intrathecal, oral, topical, or inhalation routes. Such pharmaceutical compositions can encompass pharmaceutically acceptable carriers that are inherently nontoxic and nontherapeutic. Examples of such carriers include ion exchangers, alumina, aluminum stearate, lecithin, serum proteins, such as human serum albumin, buffers such as phosphate or glycine, sorbic acid, potassium sorbate, partial glyceride mixtures of saturated vegetable fatty acids, water, salts, or electrolytes such as protamine sulfate, sodium chloride, metal salts, colloidal silica, magnesium trisilicate, polyvinyl pyrrolidone, cellulosic polymers, and polyethylene glycol.

The invention is now further illustrated by means of examples.

### Example 1: Production of liposomes having incorporated into their membrane cell adhesion molecules

Dry 0.5 µmol of dioleoyl phosphatidyl choline under nitrogen in a disposable glass tube. Evacuate in dessicator under vacuum for 30 minutes. Add buffer/dH2O to required volume and scrape the sides of the glass tube to dislodge the lipid. Add protein a 1µg/µl of lipid used. Vortex for 30 seconds. Sonicate twice in a bath sonicator at 7 degree for 15 seconds.

This procedure results in multilamellar vesicles that become small unilamellar vesicles (SUV) with prolonged sonication time. To make large unilamellar vesicles, use the extruder.

### Example 2: Expression of lipid tagged proteins and preparation of liposomes having incorporated said lipid tagged proteins.

E. coli strain HB101 was transformed with a plasmid and cultured in LB medium with ampicilin at 37°C. The plasmid encodes a hexahistidinyl tail for purification of the tagged protein. The DNA sequence encoding the signal peptide and the N-terminal amino acid residues of the major lipoprotein of E. coli (lpp) was linked to the DNA encoding the protein. The N-terminal cysteine residue of the resulting protein is enzymatically linked with the lipids in the bacterial membrane. After induction with IPTG, the cells were cultured another 12 h at 30°C and harvested by centrifugation. The cells from 11 culture were suspended in 50 ml lysis buffer and lysed by sonication. The cell envelopes were collected by ultracentrifugation (150'000 g, 1h, 4°C), the pellet was suspended in HEPES buffer (pH 7.4) containing 1% (w/v) Triton-X-100. The sample was applied to a Ni²⁺ column to purify the lipid tagged proteins having a hexahistidinyl tail.

Ten milligrams of phosphatidylcholine was dissolved in 1 ml of chloroform in a test tube. After drying well under a stream of nitrogen, it was suspended in 1 ml HEPES buffer solution (pH 7.4) and sonicated for 10 min. After centrifugation at 30'000 g for 20 min, the final pellet of liposomes was suspended in 1 ml of HEPES buffer. Then the solution of purified lipid tagged proteins were added to the resulting liposomes with stirring at 4°C.

## Claims

1. A delivery system for pharmaceutical agents wherein said system comprises liposomes which comprise in their internal compartment a pharmaceutical agent and which have linked to their external surface the cell adhesion molecule NCAM or a fragment thereof.

2. The delivery system of claim 1, wherein said NCAM fragment comprises IG loop domains I, II and III.

3. The delivery system according to claim 1 or 2, wherein said cell adhesion molecule is linked to said external surface of said liposomes via a transmembrane domain or a hydrophobic anchor molecule.

4. The delivery system according to anyone of the preceding claims, wherein said pharmaceutical agent is selected from the group consisting of DNA, RNA, oligonucleotides, polypeptides, peptides, antineoplastic agents, hormones, vitamins, enzymes, antivirals, antibiotics, antiinflammatories, antiprotozoans, antirheumatics, radioactive compounds, antibodies, prodrugs, and combinations thereof.

5. The delivery system according to claim 4, wherein said pharmaceutical agent is a DNA, preferably a cDNA which is operably linked to a gene expression construct.

6. The delivery system according to claim 5, wherein said cDNA encodes a functional protein.

7. The delivery system according to claim 6, wherein said functional protein is the human dystrophin protein.

8. The delivery system according to anyone of claims 5 to 6, wherein said delivery system comprises a DNA compacting agent.

9. The delivery system according to claim 8, wherein said DNA compacting agent is a reversibly cross-linkable cation.

10. The delivery system according to claim 9, wherein said reversible cross-link is a thio bridge.

11. The delivery system according to anyone of claims 5-10, wherein said delivery system comprises a chemical inclusion and/or a biological inclusion for the breaching of the endosomal barrier.

12. The delivery system according to anyone of claims 5-11, wherein said delivery system comprises a nuclear localisation signal, preferably in the form of PNA linked peptides or PNA linked ligands.

13. The delivery system according to anyone of claims 5-12, wherein said delivery system comprises an anti-apoptotic activity.

14. The delivery system according to claim 13, wherein said anti-apoptotic activity is selected from the group consisting of Bcl-2, a small interfering RNA directed against Bax, a peptide comprising caspase inhibitor sequences, preferably Bcl XL.

15. The delivery system according to anyone of claims 4 to 14, wherein said delivery system comprises a DNA integrase activity or a molecule encoding such a DNA integrase activity.

16. The delivery system according to claim 15, wherein said integrase is the integrase of phi C31 bacteriophage.

17. A pharmaceutical composition comprising a delivery system according to anyone of caims 1 to 16.

## Patentansprüche

1. Ein Abgabesystem für pharmazeutische Mittel, worin das System Liposomen umfasst, welche in ihrem Innenraum ein pharmazeutisches Mittel beinhalten und welche an ihrer äusseren Oberfläche das Zelladhäsionsmolekül NCAM oder Fragmente davon gebunden haben.

2. Das Abgabesystem gemäss Anspruch 1, worin das NCAM-Fragment die IG loop Domänen I, II und III beinhaltet.

3. Das Abgabesystem gemäss Anspruch 1 oder 2, worin das Zelladhäsionsmolekül an der der äusseren Oberfläche des Liposoms über eine Transmembrandomäne oder ein hydrophobes Ankermolekül gebunden ist

4. Das Abgabesystem gemäss irgendeinem der vorhergehenden Ansprüche, worin das pharmazeutische Mittel ausgewählt ist aus der Gruppe bestehend aus DNA, RNA, Oligonukleotiden, Polypeptiden, Peptiden, antineoplastische Mitteln, Hormonen, Vitaminen, Enzymen, antivirale Mitteln, Antibiotika, antiinflammatorischen Mitteln, Antiprotozoen, Antirheumatika, radioaktiven Substanzen, Antikörpern, Pro-Pharmaka, sowie Kombinationen davon.

5. Das Abgabesystem gemäss Anspruch 4, worin das pharmazeutische Mittel DNA ist, vorzugsweise eine cDNA, welche funktionsfähig mit einem Genexpressionskonstrukt verbunden ist.

6. Das Abgabesystem gemäss Anspruch 5, worin die cDNA ein funktionelles Protein kodiert.

7. Das Abgabesystem gemäss Anspruch 6, worin das funktionelle Protein das humane Dystrophinprotein ist.

8. Das Abgabesystem gemäss irgendeinem der Ansprüche 5 bis 6, worin das Abgabesystem ein DNA-Kompaktierungsmittel beinhaltet.

9. Das Abgabesystem gemäss Anspruch 8, worin das DNA-Kompaktierungsmittel ein reversibel quervernetzbares Kation ist.

10. Das Abgabesystem gemäss Anspruch 9, worin die reversible Quervernetzung eine Disulfidbrücke ist.

11. Das Abgabesystem gemäss irgendeinem der Ansprüche 5-10, worin das Abgabesystem einen chemischen Einschluss (Inklusion) und/oder einen biologischen Einschluss zum Übertreten der endosomalen Barriere beinhaltet.

12. Das Abgabesystem gemäss irgendeinem der Ansprüche 5-11, worin das Abgabesystem ein nukleäres Lokalisationssignal beinhaltet, vorzugsweise in Form von PNA gebundenen Peptiden oder PNA gebundenen Liganden.

13. Das Abgabesystem gemäss irgendeinem der Ansprüche 5-12, worin das Abgabesystem eine antiapoptotische Aktivität beinhaltet.

14. Das Abgabesystem gemäss Anspruch 13, worin die antiapoptotische Aktivität ausgewählt ist aus der Gruppe bestehend aus Bcl-2, einer kurzen interferierenden gegen Bax gerichteten RNA, einem Peptid umfassend Caspase-Inhibitorsequenzen, vorzugsweise Bcl XL.

15. Das Abgabesystem gemäss irgendeinem der Ansprüche 4 bis 14, worin das Abgabesystem eine DNA Integrase-Aktivität umfasst oder ein Molekül, welches eine solche DNA Integrase-Aktivität kodiert.

16. Das Abgabesystem gemäss Anspruch 15, worin die Integrase die Integrase des Bakteriophagen phi C31 ist.

17. Eine pharmazeutische Zusammensetzung umfassend ein Abgabesystem gemäss irgendeinem der Ansprüche 1 bis 16.

## Revendications

1. Système d'administration d'agents pharmaceutiques, dans lequel le système comprend des liposomes qui comprennent, dans leur compartiment interne, un agent pharmaceutique et qui ont, liée à leur surface extérieure, la molécule d'adhésion cellulaire NCAM ou l'un de ses fragments.

2. Système d'administration suivant la revendication 1, dans lequel le fragment de NCAM comprend des domaines I, II et III de boucle IG.

3. Système d'administration suivant la revendication 1 ou 2, dans lequel la molécule d'adhésion cellulaire est reliée à la surface extérieure des liposomes par un domaine transmembranaire ou par une molécule hydrophobe d'ancrage.

4. Système d'administration suivant l'une quelconque des revendications précédentes, dans lequel l'agent pharmaceutique est choisi dans le groupe consistant en un ADN, un ARN, les oligonucléotides, les polypeptides, les peptides, les agents antinéoplastiques, les hormones, les vitamines, les enzymes, les antiviraux, les antibiotiques, les antiinflammatoires, les antiprotozoans, les antirhumatismaux, les composés radioactifs, les anticorps, les promédicaments et leurs combinaisons.

5. Système d'administration suivant la revendication 4, dans lequel l'agent pharmaceutique est un ADN, de préférence un ADNc, qui est relié fonctionnellement à un produit d'assemblage d'expression de gène.

6. Système d'administration suivant la revendication 5, dans lequel l'ADNc code une protéine fonctionnelle.

7. Système d'administration suivant la revendication 6, dans lequel la protéine fonctionnelle est la protéine de dystrophine humaine.

8. Système d'administration suivant l'une quelconque des revendications 5 à 6, dans lequel le système d'administration comprend un agent de compactage d'ADN.

9. Système d'administration suivant la revendication 8, dans lequel l'agent de compactage d'ADN est un cation réticulable de manière réversible.

10. Système d'administration suivant la revendication 9, dans lequel la réticulation réversible est un pont thio.

11. Système d'administration suivant l'une quelconque des revendications 5 à 10, dans lequel le système d'administration comprend une inclusion chimique et/ou une inclusion biologique pour la rupture de la barrière endosomale.

12. Système d'administration suivant l'une quelconque des revendications 5 à 11, dans lequel le système d'administration comprend un signal de localisation nucléaire, de préférence sous la forme de peptides à liaison APN ou de ligands à liaison APN.

13. Système d'administration suivant l'une quelconque des revendications 5 à 12, dans lequel le système d'administration comprend une activité anti-apoptotique.

14. Système d'administration suivant la revendication 13, dans lequel l'activité anti-apoptotique est choisie dans le groupe consistant en Bcl-2, un petit ARN interférant dirigé contre Bax, un peptide comprenant des séquences d'inhibiteur de caspase, de préférence Bcl XL.

15. Système d'administration suivant l'une quelconque des revendications 4 à 14, dans lequel le système d'administration comprend une activité d'intégrase d'ADN ou une molécule codant une activité d'intégrase d'ADN de ce genre.

16. Système d'administration suivant la revendication 15, dans lequel l'intégrase est l'intégrase du bactériophage phi C31.

17. Composition pharmaceutique comprenant un système d'administration suivant l'une quelconque des revendications 1 à 16.
